# EUROPEAN PATENT APPLICATION

(11) **EP 1 522 589 A1**
(43) Date of publication of application: **13.04.2005**
(21) Application number: 04027244.5
(22) Date of filing: 15.10.2001
(51) Int. Cl.: C12P 7/62, A61K 31/365

(54) **Composition comprising pravastatin**

(30) Priority: 16.10.2000 JP 2000315256
(62) Divisional of application: 01974858.1
(71) Applicant: Sankyo Co., Ltd., Tokyo 103-8426 (JP)
(72) Inventor: Sugio, Nobunari, Iwaki-shi Fukushima 971-8183 (JP); Kojima, Shunshi, Hiratsuka-shi Kanagawa 254-0014 (JP); Takamatsu, Yasuyuki, Iwaki-shi Fukushima 971-8183 (JP); Hagisawa, Minoru, Hiratsuka-shi Kanagawa 254-0014 (JP); Hamano, Kiyoshi, Shinagawa-ku Tokyo 140-8710 (JP); Suzuki, Mutsuo, Hiratsuka-shi Kanagawa 254-0014 (JP)
(74) Representative: Gibson, Christian John Robert

(57) **Abstract**

A composition comprising pravastatin sodium which is industrially produced and contains a compound of formula (I) the quantity of which is 0.1 % or less by weight of the quantity of pravastatin sodium.

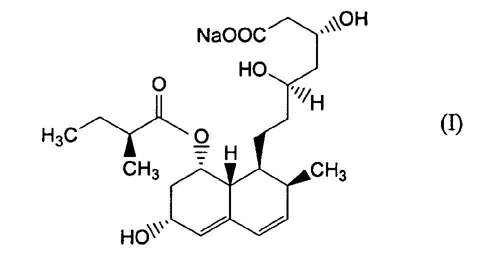

## Description

### Technical Field

The present invention relates to a process for the isolation or purification of pravastatin or a pharmacologically acceptable salt thereof which process comprises carrying out a step of extracting pravastatin and analogues thereof using an organic solvent having formula CH₃CO₂R (wherein R represents an alkyl group having three or more carbons); relates to a process for the isolation or purification of pravastatin or a pharmacologically acceptable salt thereof which process comprises performing a step comprising the decomposition of impurities using an inorganic acid; and relates to a process for the isolation or purification of pravastatin or a pharmacologically acceptable salt thereof which process comprises performing a step comprising the decomposition of impurities using an inorganic base.

Further, the present invention relates to a process for isolating or purifying pravastatin or a pharmacologically acceptable salt thereof which combines two or more steps selected from the above-mentioned three steps.

Further, the present invention relates to a composition comprising pravastatin sodium which is industrially produced, wherein a compound having formula (I) is contained in an amount of 0.1% or less by weight of pravastatin sodium.

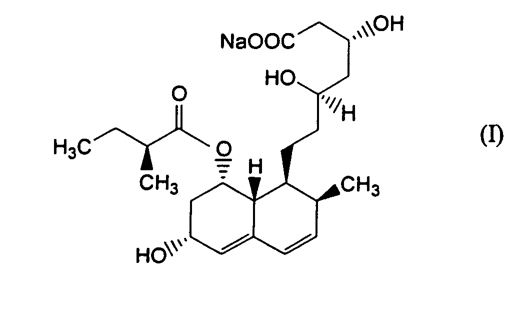

### Background Art

Pravastatin is a compound which is disclosed as a HMG-CoA reductase inhibitor in Japanese Patent Application publication (Kokai) No. Sho 57-2240 (USP 4,346,227) and has the following formula (II), and pravastatin sodium is commercially available as a therapeutic agent for hyperlipidemia at present.

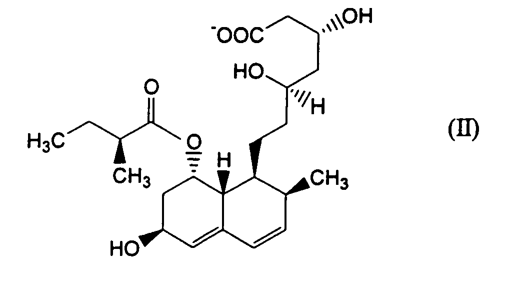

Many compounds in addition to pravastatin are known as HMG-CoA reductase inhibitors, for example, atorvastatin, fluvastatin, itavastatin and the like are compounds which are produced synthetically.

On the other hand, although lovastatin and simvastatin are produced by fermentation similarly to the case of pravastatin, they are prepared by one-stage fermentation, which differs from pravastatin in that it is prepared by a two-stage fermentation. Namely, pravastatin sodium is prepared by microbial transformation in a second step of a precursor which was obtained in a first step fermentation.

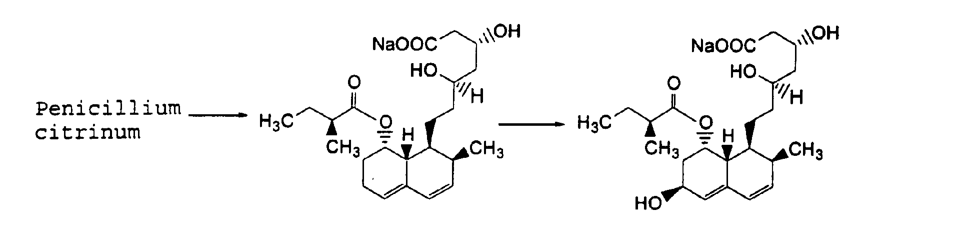

In the first place, when the microorganism is fermented, much more unexpected impurities are apt to be produced than the case of carrying out synthesis by a chemical reaction. Even though purification is respectively carried out in every step, it is not possible to remove all of the impurities completely and, in particular, it is especially difficult to remove the impurities in a large scale cultivation for industrial production.

On the other hand, "one of the important factors for producing a safe and effective pharmaceutical is to obtain a highly pure product. A chemical substance is obtained by production processes such as a chemical synthesis from a starting material, the isolation or purification of said substance produced by an organism, and the isolation or purification of said substance prepared by a production utilizing genetic recombination of a cell. In general, even if any production process including a genetic recombination process is adopted, it is often difficult to obtain a produced chemical substance having a purity of 100% because of the purity of the starting materials, the incompleteness of the reactions, decomposition in isolation or purification steps, and the like. Further, in the field of pharmaceuticals, it is clear that it is apparently common knowledge in the technical field to which the invention of the present application belongs that diagnostic agents and therapeutic agents containing impurities may have an undesirable effect on diagnosis and therapy. In conclusion, it is important to obtain a product being as highly pure as possible." [Tokyo High Court: the 302^{nd} case in Heisei 9 (Gyo-ke); (Judgment on Feb. 17, 2000)].

Furthermore, the HMG-CoA reductase inhibitor is a pharmaceutical whose administration period is long in order to effectively lower the cholesterol level in blood, therefore it is required that it is particularly high in purity in order to reduce any adverse reactions to the utmost.

Accordingly, as described above, since pravastatin which is prepared by two-step fermentation contains more impurities than simvastatin and lovastatin which are prepared by one-step fermentation, the purification step is particularly important, and studies have continued to find processes for isolation or purification of the highly pure pravastatin by removing the impurities.

The present inventors have particularly found that when pravastatin is produced by the two-step fermentation, the following compound (I) is always co-produced, 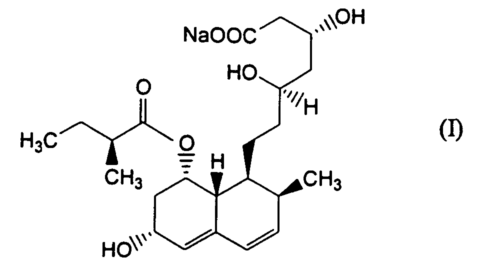 and further, the amount produced of the above-mentioned compound (I) is the highest among the impurities which are formed during microbial transformation to give pravastatin. However, although the present inventors have found that it is an important subject to strictly remove the above-mentioned compound (I) from the impurities produced during pharmaceutical production, in order to obtain highly pure pravastatin, it has been very difficult to remove the above-mentioned compound (I) in comparison with other impurities because it is the optical isomer regarding a hydroxy group at one of the asymmetric carbons of pravastatin.

Accordingly, a process has been required which is not troublesome and industrially disadvantageous such as chromatography, does not damage the productivity of the steps, and results in isolation or purification of pravastatin or its pharmacologically acceptable salt to a purity which is as high as that obtained when it is purified by chromatography; and further, a simple isolation or purification process which can remove the above-mentioned compound (I) and obtain highly pure pravastatin.

On the other hand, the following processes are known as processes for purifying the HMG-CoA reductase inhibitor.

### (1) WO92/16276 Publication (Japanese Patent Application Publication No. (Kohyo) Hei 6-506210)

This Publication relates to "a process for purifying a HMG-CoA reductase inhibitor which uses high performance liquid chromatography, and HMG-CoA reductase inhibitors obtained by the purification process".

The purification process of the publication uses high performance liquid chromatography in the purification step of the HMG-CoA reductase inhibitor. On the other hand, isolation or purification process of the present invention differs from the purification process of the publication, from the viewpoint that it provides highly pure pravastatin without using high performance liquid chromatography, by removing the impurities of pravastatin using an organic solvent which has a formula CH₃CO₂R (wherein R represents an alkyl group having three or more carbons) such as n-propyl acetate or n-butyl acetate, and/or removing impurities by decomposing them using an inorganic acid and/or an inorganic base.

Further, the isolation or purification process of the present invention "which process comprises removing compound (I) to an amount of 0.1% or less by weight of pravastatin sodium" is not described nor suggested.

By the way, since high performance liquid chromatography requires large quantities of solvents and small quantities of involatile impurities present therein, this becomes more serious in the case of a large scale production. When the solvents are removed, the samples will be contaminated with the involatile impurities. Additionally, serious environmental pollution and a large amount of distillation expense result from the use of a large quantity of solvents.

Accordingly, high performance liquid chromatography is troublesome and industrially disadvantageous, and it cannot be considered to be a process which is suitable for the industrial isolation or purification of pravastatin.

Further, even if high performance liquid chromatography is used for the industrial production of pravastatin, it is difficult to remove the above-mentioned compound (I) which is the optical isomer of pravastatin and to obtain pravastatin having the desired purity.

### (2) WO99/42601 Publication

This Publication relates to "an isolation or purification process by which a concentrated cultured broth containing a HMG-CoA reductase inhibitor is adjusted to from pH 4.5 to 7.5 with an acid, then the HMG-CoA reductase inhibitor is extracted with ethyl acetate, and after lactonization, if desired, it is crystallized to obtain the HMG-CoA reductase inhibitor having a purity of 99.6% or more".

The purification process of the publication differs from the isolation or purification process of the present invention, from the viewpoint that the purification process of the publication uses ethyl acetate as an organic solvent in a step of extracting pravastatin and analogues thereof from a concentrated cultured broth which contains pravastatin and analogues thereof produced by a microorganism, whereas in contrast the purification process of the present invention uses an organic solvent which has the formula CH₃CO₂R (wherein R represents an alkyl group having three or more carbons) such as n-propyl acetate or n-butyl acetate.

Further, the isolation or purification process of the present invention affords highly pure pravastatin, by carrying out a step of decomposing the impurities of pravastatin using an inorganic acid and/or an inorganic base, but a step of decomposing the impurities of the HMG-CoA reductase inhibitor using an inorganic acid and/or an inorganic base is not described nor suggested at all in the publication.

Further, the isolation or purification process of the present invention "which process comprises removing compound (I) to an amount of 0.1% or less by weight of pravastatin sodium" is not described nor suggested at all.

Certainly, an isolation or purification process of pravastatin is described in Example 3 of the Publication. However, in this Example, the purity of pravastatin which was extracted with ethyl acetate remains only at 70.3% or less, and then, although there is a description of purifying it using chromatography which differs from the isolation or purification process of the present invention, the purity of pravastatin finally obtained is not described at all. Accordingly, it is not obvious whether highly pure pravastatin is finally obtained or not, and additionally, it is not suggested that the above-mentioned compound (I) is purified to an amount of 0.1% or less by weight of pravastatin.

Further, it is difficult to believe that even lovastatin described to be obtained at a high purity of 99.6% or more in an Example of the publication is truly obtained at this high purity of 99.6% or more judging from the HPLC chart (Fig.4) of the final product, and it has no credibility.

### (3) WO00/17182 Publication

This Publication relates to "a purification process for a HMG-CoA reductase inhibitor which uses displacement chromatography and a HMG-CoA reductase inhibitor obtained by the purification process".

The purification process of the publication comprises using displacement chromatography in a step for purifying the HMG-CoA reductase inhibitor. On the other hand, the isolation or purification process of the present invention differs from the purification process of the publication, from the viewpoint that the isolation or purification process of the present invention affords highly pure pravastatin without using any chromatography including displacement chromatography, by removing the impurities of pravastatin by using an organic solvent which has the formula CH₃CO₂R (wherein R represents an alkyl group having three or more carbons) such as n-propyl acetate or n-butyl acetate, and/or removing impurities by decomposing them using an inorganic acid and/or an inorganic base.

Further, the isolation or purification process of the present invention "which process comprises removing compound (I) to an amount of 0.1% or less by weight of pravastatin sodium" is not described nor suggested at all.

Furthermore, even if displacement chromatography is used for the industrial production of pravastatin, it is troublesome, and it is difficult to remove the above-mentioned compound (I) which is the optical isomer of pravastatin.

Further, isolation or purification processes other than the above-mentioned purification processes can be adopted by those skilled in the art, for example analytical high pressure liquid chromatography (HPLC) can be mentioned, but analytical HPLC is not a practical procedure as an isolation or purification process for pravastatin. Analytical HPLC uses a packing column (a diameter of 4.6 mm x a length of 25 cm) having a very narrow diameter, about 10 µg (1 x 10⁻² mg) of a sample is added to a highly-pressured column at every analysis, and one analysis requires about 30 minutes. Although the sample flows from the column, it is an extremely diluted solution, and although detection of components can be made in the analytical system, collection is not practical. Further, a collection system is not attached to the analytical HPLC, and even if it is installed, the removal of solvent is required when a solid sample is desired to be obtained. When analytical HPLC is used for the isolation or purification of pravastatin, 500 runs of analyses and 250 hours are required for isolating or purifying pravastatin needed for making merely one 5 mg tablet, and analytical HPLC is an unrealistic process for isolation or purification of pravastatin. Therefore, it is clear that analytical HPLC is industrially disadvantageous.

Further, preparative HPLC cannot be said to be a procedure suitable for the industrial scale isolation or purification of pravastatin.

Silica gel chromatography can be roughly classified into column chromatography and flash chromatography based on the size of the silica gel particles, but it is not expected to be a procedure suitable for use in an industrial scale isolation or purification.

The above-mentioned compound (I) and other impurities have actually been tried to be separated using various chromatographies, but compound (I) could not be separated from pravastatin because the above-mentioned compound (I) is the stereoisomer of pravastatin.

Recrystallization is an isolation or purification process which is widely used in the field of pharmaceuticals. However, although recrystallization has been used, it has not been effective for selective isolation of the above-mentioned compound (I) which resembles pravastatin in structure, from pravastatin and for improving the purity of pravastatin in a composition to a desired level.

Although other isolation or purification processes such as gas chromatography and distillation have been considered, both of them are separation procedures based on the size difference of sample molecules, and both require heating of samples. However, since pravastatin is apt to be decomposed at its melting point, these cannot be used as the isolation or purification process.

Further, even if either of the above-mentioned isolation or purification processes is repeated for removing the above-mentioned compound (I), the yield of pravastatin is resultantly decreased.

Further, even if any of the conventionally known isolation or purification processes described above are used in combination, they could not be a process for removing the above-mentioned compound (I) to an amount of 0.1% or less by weight and being applied for the industrial production of pravastatin.

Accordingly, there has not been known at all an isolation or purification process which can provide a highly pure pravastatin by removing, in particular, the compound having the above-mentioned formula (I) which is a substance related to pravastatin without lowering the productivity of the step.

### DISCLOSURE OF THE INVENTION

The present inventors have intensively studied a pharmaceutical composition containing pravastatin, and as a result, have found an isolation or purification process by which highly pure pravastatin (preferably having a purity of 99.5% or more) is obtained, further, an isolation or purification process for removing impurities which include the above-mentioned compound (I) which is an analogue of pravastatin, from said pharmaceutical composition, and in particular, an isolation or purification process for removing the above-mentioned compound (I) to an amount of 0.1% or less by weight of pravastatin, and have completed the present invention.

The present invention relates to:
(1) A process for isolation or purification of pravastatin or a pharmacologically acceptable salt thereof which process comprises performing an extraction of pravastatin and analogues thereof with an organic solvent having the formula CH₃CO₂R (wherein R represents an alkyl group having three or more carbons);
(2) A process for the isolation or purification ofpravastatin or a pharmacologically acceptable salt thereof which process comprises decomposing impurities using an inorganic acid; and
(3) A process for the isolation or purification of pravastatin or a pharmacologically acceptable salt thereof which process comprises decomposing impurities using an inorganic base;
   preferably relates to:
(4) A process for the isolation or purification of pravastatin or a pharmacologically acceptable salt thereof according to (1) wherein R is a straight or branched chain alkyl group having 3 or 4 carbons;
(5) A process for the isolation or purification of pravastatin or a pharmacologically acceptable salt thereof according to (1) wherein R is an-propyl or n-butyl group;
(6) A process for the isolation or purification of pravastatin or a pharmacologically acceptable salt thereof according to (2) wherein said inorganic acid is phosphoric acid; and
(7) A process for the isolation or purification of pravastatin or a pharmacologically acceptable salt thereof according to (2) or (6) wherein the pH in the decomposition step using an inorganic acid is in the range from 2 to 5.
   Furthermore the present invention relates to:
(8) A process for the isolation or purification of pravastatin or a pharmacologically acceptable salt thereof which process comprises performing a combination of an extraction of pravastatin and analogues thereof with an organic solvent having the formula CH₃CO₂R (wherein R represents an alkyl group having three or more carbons) and a decomposition of impurities using an inorganic acid;
(9) A process for the isolation or purification of pravastatin or a pharmacologically acceptable salt thereof which process comprises performing a combination of a decomposition of impurities using an inorganic acid and decomposition of impurities using an inorganic base; and
(10) A process for the isolation or purification of pravastatin or a pharmacologically acceptable salt thereof which process comprises performing a combination of an extraction of pravastatin and analogues thereof with an organic solvent having the formula CH₃CO₂R (wherein R represents an alkyl group having three or more carbons); a decomposition of impurities using an inorganic acid; and a decomposition of impurities using an inorganic base;
   preferably relates to
(11) A process for the isolation or purification of pravastatin or a pharmacologically acceptable salt thereof according to (8) or (10) wherein R is a straight or branched chain alkyl group having 3 or 4 carbons;
(12) A process for the isolation or purification of pravastatin or a pharmacologically acceptable salt thereof according to (8) or (10) wherein R is a n-propyl or n-butyl group;
(13) A process for the isolation or purification of pravastatin or a pharmacologically acceptable salt thereof according to any one of (8) to (12) wherein the inorganic acid is phosphoric acid; and
(14) A process for the isolation or purification of pravastatin or a
pharmacologically acceptable salt thereof according to any one of (8) to (13) wherein the pH in the decomposition step using an inorganic acid is in the range from 2 to 5.
   In addition, the present invention relates to:
(15) A process for the isolation or purification of pravastatin or a pharmacologically acceptable salt thereof which process comprises performing a combination of an extraction of pravastatin and analogues thereof with an organic solvent having the formula CH₃CO₂R (wherein R represents an alkyl group having three or more carbons) and a decomposition of impurities using an inorganic base;
   preferably relates to:
(16) A process for the isolation or purification of pravastatin or a pharmacologically acceptable salt thereof according to (15) wherein R is a straight or branched chain alkyl group having 3 or 4 carbons; and
(17) A process for the isolation or purification of pravastatin or a pharmacologically acceptable salt thereof according to (15) wherein R is a n-propyl or n-butyl group.
   In addition the present invention also relates to:
(18) A process for the isolation or purification of pravastatin sodium which process comprises removing a compound of formula (I) so that the quantity of the compound of formula (I) is reduced to 0.1% or less by weight of the quantity of pravastatin sodium by means of performing a combination of an extraction of pravastatin and analogues thereof with an organic solvent having the formula CH₃CO₂R (wherein R represents an alkyl group having three or more carbons) and a decomposition of impurities using an inorganic acid; 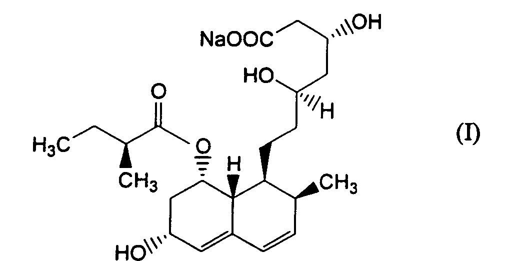
(19) A process for the isolation or purification of pravastatin sodium which process comprises removing a compound of formula (I) so that the quantity of the compound of formula (I) is reduced to 0.1% or less by weight of the quantity of pravastatin sodium by means of performing a combination of a decomposition of impurities with an inorganic acid and a decomposition of impurities using an inorganic base; and 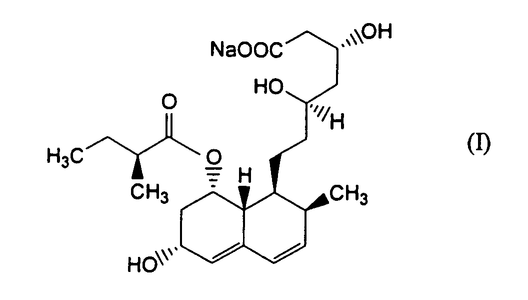
(20) A process for the isolation or purification of pravastatin sodium which process comprises removing a compound of formula (I) so that the quantity of the compound of formula (I) is reduced to 0.1% or less by weight of the quantity of pravastatin sodium by means of performing a combination of an extraction of a pravastatin and analogues thereof with an organic solvent having the formula CH₃CO₂R (wherein R represents an alkyl group having three or more carbons); a decomposition of impurities using an inorganic acid; and a decomposition of impurities using an inorganic base. 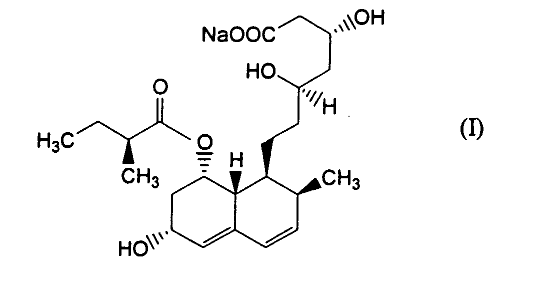
   preferably relates to:
(21) A process for the isolation or purification of pravastatin sodium according to (18) or (20) wherein R is a straight or branched chain alkyl group having 3 or 4 carbons;
(22) A process for the isolation or purification of pravastatin sodium according to claim (18) or (20) wherein R is a n-propyl or n-butyl group;
(23) A process for the isolation or purification of pravastatin sodium according to any one of (18) to (22) wherein the inorganic acid is phosphoric acid;
(24) A process for the isolation or purification of pravastatin sodium according to any one of (18) to (23) wherein the pH in the decomposition step using an inorganic acid is in the range from 2 to 5; and
(25) A process for the isolation or purification ofpravastatin sodium according to any one of (18) to (23) wherein the purity of Pravastain sodium is 99.5% or more.
   Furthermore the present invention also relates to:
(26) A process for the isolation or purification of pravastatin sodium which process comprises removing a compound of formula (I) so that the quantity of the compound of formula (I) is reduced to 0.1% or less by weight of the quantity of pravastatin sodium by means of performing a combination of an extraction of pravastatin and analogues thereof with an organic solvent having the formula CH₃CO₂R (wherein R represents an alkyl group having three or more carbons) and a decomposition of impurities using an inorganic base. 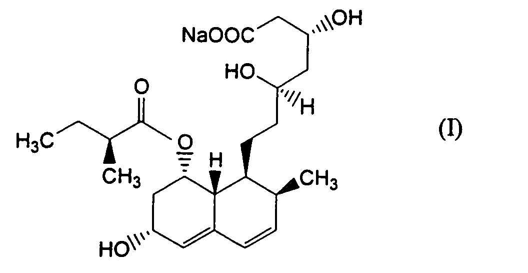
   preferably relates to:
(27) A process for the isolation or purification of pravastatin sodium according to (26) wherein R is a straight or branched chain alkyl group having 3 or 4 carbons;
(28) A process for the isolation or purification of pravastatin sodium according to (26) wherein R is a n-propyl or n-butyl group; and
(29) A process for the isolation or purification of pravastatin sodium according to any one of (26) to (28) wherein the purity of pravastatin sodium is 99.5% or more.
   Furthermore the present invention relates to:
(30) A composition comprising pravastatin sodium which is industrially produced and contains a compound of formula (I) the quantity of which is 0.1% or less by weight of the quantity of pravastatin sodium; 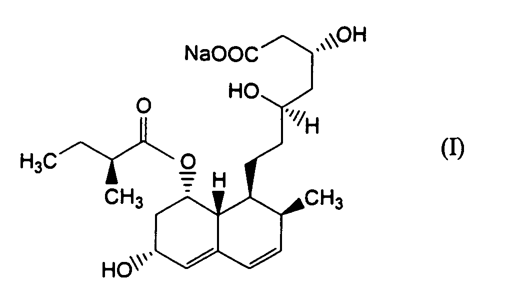
   preferably relates to:
(31) A composition comprising pravastatin sodium according to (30) which is industrially produced and contains 99.5% or more of pravastatin sodium;
(32) A composition comprising pravastatin sodium which is obtained by isolation or purification according to any one of (8) to (17) and contains a compound of formula (I) the quantity of which is 0.1% or less by weight of the quantity of pravastatin sodium; 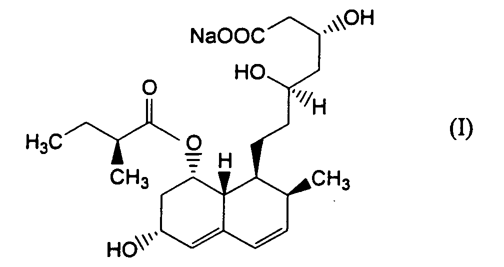
(33) A composition comprising pravastatin sodium according to (32) wherein the purity of pravastatin sodium is 99.5% or more.

In the present invention, "pravastatin and analogues thereof" means pravastatin having a formula (II) or a pharmacologically acceptable salt thereof, and compounds which have a related structure to the above-mentioned formula (II), namely a pravastatin analogue [for example, the above-mentioned compound (I) can be mentioned].

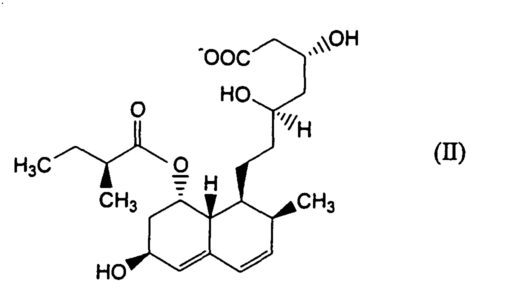

The isolation or purification of pravastatin or a pharmacologically acceptable salt thereof is typically attained by the isolation or purification processes described below.

Firstly, a cultured broth containing pravastatin obtained after terminating transformation cultivation is separated from the mycelium by a filtration and/or centrifugation method according to a conventional method, and then concentrated to obtain a concentrated culture filtrate.

The pH of the concentrated culture filtrate thus obtained is adjusted using an acid, if necessary, and then pravastatin and analogues thereof are extracted using an organic solvent such as ethyl acetate which is hardly miscible with water. For example, in WO99/42601 publication, the pH of a concentrated culture filtrate containing a HMG-CoA reductase inhibitor is adjusted to from 4.5 to 7.5 (preferably, 5.5 to 7.5) using an acid, and then is extracted with ethyl acetate.

Pravastatin and analogues thereof thus obtained are collected from the above-mentioned extract according to a conventional method. For example, the above-mentioned extract is washed with water, a saturated aqueous sodium chloride solution and the like, sodium hydroxide is added to the extract, and extraction and phase separation are carried out to obtain an aqueous solution of the salt of pravastatin as a reverse extracted aqueous layer. The salt of pravastatin obtained can be crystallized if desired.

The crystallization which is carried out according to necessity can be carried out as described below, according to the methods which are generally known in the technology of organic synthetic chemistry [for example, a process described in Ullmann's Encyclopedia of Industrial Chemistry, Vol.A24, 5th edition (1993), pp. 437-505].

As the crystallization method, for example, pravastatin can be obtained as a crystalline form by adding an organic solvent and water to the composition of pravastatin or a pharmacologically acceptable salt thereof, dissolving it by heating, and seeding with a small amount of the pravastatin salt.

The organic solvent used for crystallization is, for example, an aliphatic hydrocarbon such as hexane and heptane; an aromatic hydrocarbon such as toluene and xylene; an ester such as methyl acetate, ethyl acetate, n-propyl acetate and n-butyl acetate; an organic acid such as acetic acid; an alcohol such as methanol, ethanol, n-propanol, isopropanol, n-butanol, isobutanol, tert-butanol, isoamyl alcohol, diethylene glycol, glycerol and octanol; a ketone such as acetone and methyl ethyl ketone; an ether such as diethyl ether, di-isopropyl ether, tetrahydrofuran, dioxane, dimethoxyethane and diethyleneglycol dimethyl ether; an amide such as formamide, dimethylformamide, dimethylacetamide and hexamethylphosphoric acid triamide; a sulfoxide such as dimethyl sulfoxide; or a mixed solvent of water and one or more of the above-mentioned organic solvents. A mixed solvent of water and one or more of the above-mentioned organic solvents is preferable, a mixed solvent of water and one or more of organic solvents selected from alcohols, esters and ketones is more preferable, and a mixed solvent of water, alcohols and esters is most preferable.

The isolation or purification process of the present invention starts with the concentrated culture filtrate obtained according to a conventional method, and then extracts it, not using ethyl acetate but using an organic solvent having the formula CH₃CO₂R (wherein R represents an alkyl group having three or more carbons) in the step of isolating or purifying pravastatin or a pharmacologically acceptable salt thereof.

The "alkyl group having three or more carbons" in the definition of R in the above-mentioned formula is for example, a group such as a n-propyl group, an isopropyl group, a n-butyl group, an isobutyl group, a sec-butyl group, a tert-butyl group, a pentyl group, an isopentyl group, a 2-methylbutyl group, a neopentyl group, a 1-ethylpropyl group, a hexyl group, an isohexyl group, a 4-methylpentyl group, a 3-methylpentyl group, a 2-methylpentyl group, a 1-methylpentyl group, a 3,3-dimethylbutyl group, a 2,2-dimethylbutyl group, a 1,1-dimethylbutyl group, a 1,2-dimethylbutyl group, a 1,3-dimethylbutyl group, a 2,3-dimethylbutyl group, a 1-ethylbutyl group and a 2-ethylbutyl group. A straight or branched chain alkyl group having 3 to 6 carbons is preferable, a straight or branched chain alkyl group having 3 or 4 carbons is further preferable, and a n-propyl group or a n-butyl group is most preferable.

Further, the isolation or purification process of the present invention is also characterized in comprising a step of decomposing impurities of pravastatin using an inorganic acid, and/or a step of decomposing impurities using an inorganic base, in the isolation or purification process of the above-mentioned pravastatin.

When two or more of the steps selected from the step of extracting pravastatin using an organic solvent having the above-mentioned formula CH₃CO₂R, the step of decomposing the impurities present with pravastatin using an inorganic acid, and the step of decomposing impurities using an inorganic base, are used in combination, the desired steps can be appropriately performed randomly in the isolation or purification process. Specific examples of the isolation or purification process include:
(a) a process for the isolation or purification of pravastatin or a pharmacologically acceptable salt thereof which comprises the combination of a step of extracting pravastatin and analogues thereof using an organic solvent having the formula CH₃CO₂R (wherein R represents an alkyl group having three or more carbons) and the step of decomposing the impurities using an inorganic acid,
(b) a process for the isolation or purification of pravastatin or a pharmacologically acceptable salt thereof which comprises a combination of a step of extracting pravastatin and analogues thereof using an organic solvent having the formula CH₃CO₂R (wherein R represents an alkyl group having three or more carbons) and the step of decomposing impurities using an inorganic base,
(c) a process for the isolation or purification of pravastatin or its pharmacologically acceptable salt which comprises a combination of a step of decomposing impurities using an inorganic acid and a step of decomposing impurities using an inorganic base, and
(d) a process for the isolation or purification of pravastatin or a pharmacologically acceptable salt thereof which comprises a combination of a step of extracting pravastatin and analogues thereof using an organic solvent having the formula CH₃CO₂R (wherein R represents an alkyl group having three or more carbons), the step of decomposing impurities using an inorganic acid and a step of decomposing impurities using an inorganic base.

The step of decomposing the impurities of pravastatin or a pharmacologically acceptable salt thereof using an inorganic acid is carried out in the presence or absence of an inert solvent (preferably, in the presence) under conditions in which the pH of the solution of pravastatin or a pharmacologically acceptable salt thereof is adjusted to 2 to 5 (preferably 3 to 4).

The "inorganic acid" which is used in the decomposition of impurities by an inorganic acid is not specifically limited, so long as it is used as an inorganic acid in a usual reaction, but for example, inorganic acids such as hydrobromic acid, hydrochloric acid, sulfuric acid, perchloric acid, phosphoric acid, and nitric acid can be used. Phosphoric acid or sulfuric acid are preferable, and phosphoric acid is most preferable.

The reaction temperature and the reaction time in the decomposition of impurities by an inorganic acid depend on the pH value. Basically, when the reaction temperature is low, the required reaction time is to be long, and when the reaction temperature is high, the required reaction time is to be short. For example, the reaction temperature is 20°C to 80°C (preferably, 40°C to 60°C), and the reaction time is one minute to 6 hours (preferably, 5 minutes to 20 minutes).

While the "inert solvent" used in the decomposition of impurities by the inorganic acid is not specifically limited so long as it is used as a solvent, examples include alcohols such as methanol, ethanol, n-propanol, isopropanol, n-butanol, isobutanol, tert-butanol, isoamyl alcohol, diethylene glycol, glycerol, octanol, cyclohexanol and methylcellosolve; water; or a mixed solvent of water with the above-mentioned alcohols, preferably water or a mixed solvent of water with the above-mentioned alcohols, and most preferably water or a mixed solvent of water with ethanol.

After the reaction, pravastatin which is the desired compound is collected from the reaction solution according to a conventional method. For example, an organic solvent such as ethyl acetate which is not miscible with water is added, the organic solvent layer containing the desired compound is separated, the solvent layer is washed with water and the solvent is evaporated to obtain the desired compound. Further, if necessary, the solvent layer is decolorized by adding activated charcoal, the activated charcoal is removed by filtration, then, a reagent which forms a salt of pravastatin such as sodium hydroxide, sodium methoxide or sodium ethoxide is added, and the layer is concentrated under reduced pressure by a rotary evaporator and the like to obtain the salt of pravastatin.

The step of decomposing the impurities of pravastatin or its pharmacologically acceptable salt using an inorganic base is carried out in the presence or absence of an inert solvent (preferably, in the presence) under conditions in which the pH of the solution of pravastatin or a pharmacologically acceptable salt thereof is adjusted to 10 to 14.

The "inorganic base" which is used in the decomposition of impurities by an inorganic base is not specifically limited, so long as it is used as an inorganic base in a usual reaction. Examples include alkali metal carbonates such as lithium carbonate, sodium carbonate and potassium carbonate; alkali metal bicarbonates such as lithium hydrogencarbonate, sodium hydrogencarbonate and potassium hydrogencarbonate; alkali metal hydrides such as lithium hydride, sodium hydride and potassium hydride; alkali metal hydroxides such as lithium hydroxide, sodium hydroxide and potassium hydroxide; alkali metal alkoxides such as lithium methoxide, sodium methoxide, sodium ethoxide and potassium tert-butoxide, preferably alkali metal hydroxides, and most preferably sodium hydroxide.

The reaction temperature and the reaction time in the decomposition of impurities by the inorganic base depend on the pH value. Basically, when the reaction temperature is low, the required reaction time is to be long, and when the reaction temperature is high, the required reaction time is to be short. For example, the reaction temperature is at -10°C to 110°C, and the reaction time is for 15 minutes to 200 hours.

The "inert solvent" used in the decomposition of impurities by the inorganic base is not specifically limited, so long as it is inert for the present reaction, but similar solvents to the inert solvent used in the decomposition of impurities by the inorganic acid can be used.

As the preferable conditions for decomposing impurities by an inorganic base which are contained in the concentrated culture filtrate containing pravastatin produced by a microorganism, the pH is 11 to 14 (more preferably, 11 to 12), the reaction temperature is at 40°C to 110°C (more preferably, 95°C to 105°C), and the reaction time is for 2 hours to 24 hours (more preferably, 2 hours to 5 hours).

On the other hand, the decomposition by an inorganic base of impurities contained in the reverse extract obtained by extraction with an aqueous alkaline solution (preferably, at a pH of 8 to 9) from the organic extract which is obtained by extraction of the concentrated culture filtrate containing pravastatin produced by a microorganism with an organic solvent under acidic conditions (preferably, at a pH of 4 to 6), is preferably conducted at a pH of 13 to 14 (more preferably, 13.5 to 14), at a reaction temperature of-10°C to 50°C (more preferably, -5°C to 5°C), and at a reaction time of 2 hours to 180 hours (more preferably, 20 hours to 50 hours, and most preferably, 25 hours to 35 hours).

After the reaction, pravastatin which is the desired compound is collected from the above-mentioned reaction solution according to a conventional method. For example, an aqueous acidic solution such as an aqueous sulfuric acid solution is added, an organic solvent such as ethyl acetate which is not miscible with water is added, the organic solvent layer containing the desired compound is separated, the solvent layer is washed with water and the like and the solvent is evaporated to obtain the desired compound. Further, if necessary, the solvent layer is decolorized by adding activated charcoal, the activated charcoal is removed by filtration, then a reagent which forms a salt such as sodium methoxide, sodium ethoxide or sodium hydroxide is added, and the layer is concentrated under reduced pressure by a rotary evaporator and the like to obtain the salt of pravastatin.

Further, the isolation or purification process of the present invention may include the step of crystallizing the salt of pravastatin obtained, and the crystallization process can be carried out according to a conventional method.

### EFFECT OF THE INVENTION

The isolation or purification process of the present invention is a process for obtaining highly pure pravastatin or a pharmacologically acceptable salt thereof (preferably having a purity of 99.5% or more) without using a process which is troublesome and industrially disadvantageous such as column chromatography.

Further, the above-mentioned compound (I) is removed to an amount of 0.1% or less by weight of pravastatin by the isolation or purification process of the present invention.

### INDUSTRIAL APPLICABILITY

When pravastatin or a pharmacologically acceptable salt thereof is used as a pharmaceutical, pravastatin itself or an appropriate mixture of pravastatin with excipients, diluents and the like which are pharmacologically acceptable can be orally administrated as, for example, tablets, capsules, granules, powders or syrups, or parenterally administrated by an injection or a suppository or the like.

Further, the analysis of the purity of the present invention can be carried out using high performance liquid chromatography (HPLC). The HPLC measurement conditions are as follows:
A: Mobile phase: 20% acetonitrile, 30% methanol, 50% TEAP buffer (0.3% triethylamine-H₃PO₄ (pH3.2));
   Detection wavelength: UV 238 nm;
   Column: Reverse phase column manufactured by Waters
   Symmetry C18 3.5 µm, φ 4.6 mm x 15 cm;
   Flow rate: 1 ml/min,
B: Mobile phase: a mixed solution of methanol: water: glacial acetic acid : triethylamine (600:400:1:1);
   Detection wavelength: UV 238 nm;
   Column: Column manufactured by ELMER OPTICS
   ERC-ODS-1262, φ 6 mm x 10 cm;
   Column temperature: 30°C;
   Flow rate: 1ml/min, or,
C: Mobile phase: a mixed solution of methanol: water: glacial acetic acid: triethylamine (450:550:1:1);
   Detection wavelength: UV 238 nm;
   Column: Column manufactured by BECKMAN
   ULTRASPHERE ODS, φ 4.6 mm x 15 cm, 5 µm;
   Column temperature: 25°C;
   Flow rate: 1.3 ml/min

Examples are shown below, and the present invention is further specifically illustrated, but the present invention is not limited to these.

### Example 1 Extraction using n-butyl acetate from concentrated culture filtrate

### (1a) Concentration of culture solution

After termination of microbial conversion, 10 L of the cultured broth containing pravastatin was adjusted to a pH of 12 by sodium hydroxide, the broth was heated to 50°C and stirred for 30 minutes. After cooling the culture solution to room temperature, 500 g of Celite 545 (trade mark) (manufactured by CELITE CORPPRATION) were added as a filtration aid to perform filtration. 3 L of water were added to the residual mycelium, suspended again and filtered. The two concentrated solutions obtained were combined to obtain 10 L of a concentrated culture filtrate.

### (1b) Extraction using n-butyl acetate

After the concentrated culture filtrate which was obtained was adjusted to pH 5.7 with 25% sulfuric acid, 5 L of n-butyl acetate were added and stirred to extract pravastatin. After the separated aqueous layer was readjusted to pH 5.7 with 75% sulfuric acid, 5 L of n-butyl acetate were added and stirred to extract it. The resulting two n-butyl acetate layers were combined, and 2 L of a saturated aqueous sodium chloride solution were added thereto and stirred. 1 L of water was added to 8 L of the extract obtained by separating the upper layer, 25% sodium hydroxide was added while stirring, the pH was adjusted to 9.5, and then the aqueous layer was separated to obtain 1 L of an aqueous solution containing pravastatin sodium. The purity of pravastatin sodium according to HPLC (condition A) was 90% or more. From the result of the present Example, it is clear that highly pure pravastatin sodium is obtained.

### Example 2 Extraction using propyl acetate from concentrated culture filtrate

A treatment similar to that in Example 1 was carried out using n-propyl acetate in place of n-butyl acetate to obtain an aqueous solution of the sodium salt of pravastatin. The purity of pravastatin sodium according to HPLC (condition A) was 85% or more. From the result of the present Example, it is clear that highly pure pravastatin sodium is obtained using n-propyl acetate.

### Example 3 Decomposition of impurities by phosphoric acid

To the aqueous solution obtained in Example 1 [the compound (I)/pravastatin sodium was 9.3% according to HPLC (condition A)] was added 350 ml of ethanol, the pH was adjusted to 3.0 with phosphoric acid, and then the mixture was stirred at 50°C for 10 minutes. The compound (I)/ pravastatin sodium was 0.9 % according to HPLC (condition A). From the result of the present Example, it is clear that compound (I) is remarkably removed by using phosphoric acid.

### Example 4 Decomposition of impurities by sulfuric acid

A treatment similar to that in Example 3 was carried out using sulfuric acid in place of phosphoric acid. The compound (I)/ pravastatin sodium was 3% according to HPLC (condition A). From the result of the present Example, it is clear that compound (I) is remarkably removed by using sulfuric acid.

### Example 5 Decomposition of impurities by sodium hydroxide, extraction and crystallization

### (5a) Decomposition of impurities by sodium hydroxide

After 500 ml of the concentrated culture filtrate of pravastatin [the compound (I)/ pravastatin sodium was 12.1% according to HPLC (condition B)] were heated to 100°C, two equivalents of sodium hydroxide were added as an aqueous solution (pH = 11.3). After the reaction mixture was stirred at 100°C for 3 hours, it was cooled, the pH was adjusted to 8.5 with an aqueous 20% sulfuric acid solution at room temperature, and an alkali-treated solution with sodium hydroxide was obtained (the content of pravastatin sodium: 56.6 g). The compound (I)/ pravastatin sodium was 0.41% according to HPLC (condition B). From the result of the present Example, it is clear that compound (I) is remarkably removed by using sodium hydroxide.

### (5b) Extraction and crystallization after decomposition of impurities by sodium hydroxide

The aqueous layer was separated in a similar manner to that in Example (1b) using n-butyl acetate to give 260 g of the alkali-treated solution (content of pravastatin sodium: 19 g) with sodium hydroxide which was obtained in (5a), and an aqueous solution of pravastatin sodium was obtained.

After the aqueous solution of pravastatin soldium was concentrated to about half its amount under reduced pressure, 77 ml of water were added and the solution was concentrated again under reduced pressure. The liquid amount was adjusted with water so that the volume of concentrated solution became 6-fold relative to the weight of pravastatin (free form), and the mixture was cooled to 0 to 5°C, an aqueous 20% sulfuric acid solution was added dropwise, the free crystalline form of pravastatin was precipitated at a pH of 4.6, followed by stirring overnight and then filtering. The crystals were washed with cooled diluted sulfuric acid (pH 4) to obtain the wet crystals of pravastatin (free form).

To the wet crystals of pravastatin (free form) were added 63 ml of ethyl acetate, and after the crystals were dissolved at room temperature, the pH was adjusted to 4.5 with an aqueous 20% sulfuric acid solution while stirring at room temperature. 21 ml of water were added to the ethyl acetate layer after stirring, extraction and liquid separation, and liquid separation was performed again after extraction and washing. After 25 ml of ethanol were added to the ethyl acetate layer which was extracted and washed, the pH was adjusted to 8.7 with a 6% sodium hydroxide-ethanol solution while stirring at room temperature to obtain a sodium chloride solution. Then, it was crystallized according to a conventional method, and 8.95 g of crystals of pravastatin sodium were obtained. The purity according to HPLC (condition C) of pravastatin sodium which was obtained by this process was 99.67% and compound (I)/ pravastatin sodium was 0.1% by weight.

From the result of the present Example, it is clear that compound (I) is remarkably removed by the step of decomposing impurities by sodium hydroxide and the step of extraction using n-butyl acetate.

### Example 6 Extraction with n-butyl acetate, decomposition of impurities by phosphoric acid, and crystallization

To the acid-treated solution which was obtained in Example 3 was added an aqueous 25% sodium hydroxide solution, the pH was adjusted to 12, and the reaction solution was further stirred at 50°C for 30 minutes. The solution was concentrated to 1 L by a rotary evaporator under reduced pressure to obtain a concentrated solution which contained pravastatin sodium. After the concentrated solution obtained was adjusted to pH 4.0 with sulfuric acid, pravastatin was extracted with 0.5 L of ethyl acetate, and the extract was washed with 0.2 L of water. To the extracted solution were added 5 g of activated charcoal, the mixture was left to stand for 10 minutes, then it was filtered with a filter paper, the filtrate was adjusted to pH 8.7 with an aqueous 25% sodium hydroxide solution, and then it was evaporated to dryness by drying by a rotary evaporator under reduced pressure to obtain 50 g of pravastatin sodium. It was crystallized according to a conventional method to obtain 34 g of crude crystalline pravastatin sodium. The crude crystals were recrystallized using a solvent having the same composition, to obtain 32 g of pure crystals of pravastatin sodium.

The purity according to HPLC (condition A) of pravastatin sodium which was obtained by this process was 99.7% or more and the compound (I)/pravastatin sodium was 0.1% by weight.

From the result of the present Example, it is clear that the compound (I) is removed to 0.1% or less by weight by the step of extraction using n-butyl acetate and the step of decomposing impurities by phosphoric acid, and a highly pure pravastatin sodium was obtained.

Further, it is clear that compound (I) was further removed by adding the step of decomposing impurities by sodium hydroxide to the step of extraction using n-butyl acetate and the step of decomposing impurities by phosphoric acid, and a highly pure pravastatin sodium was obtained.

### Example 7 Decomposition of impurities by sodium hydroxide, decomposition by sulfuric acid, and crystallization

Extraction was carried out in a similar manner to that in Example (5b) using ethyl acetate from the alkali-treated solution with sodium hydroxide which was obtained in Example (5a), and an aqueous solution of pravastatin sodium was obtained. To the resulting aqueous solution were added 350 ml of ethanol, the pH was adjusted to 1.5 with sulfuric acid, followed by stirring at 20°C for one hour. The mixture was crystallized according to a conventional method to obtain pure crystals of pravastatin sodium. Compound (I)/pravastatin sodium according to HPLC (condition C) was 0.1 % or less by weight.

From the result of the present Example, it is clear that the compound (I) is removed to 0.1% or less by weight by the step of decomposing impurities by sulfuric acid and the step of decomposing impurities by sodium hydroxide, and a highly pure pravastatin sodium was obtained.

### Comparative Example 1. Extraction of pravastatin with ethyl acetate

A similar treatment to that in Example (1a) was carried out using ethyl acetate in place of n-butyl acetate to obtain an aqueous solution ofpravastatin sodium. The purity of the sodium salt of pravastatin according to HPLC (condition A) was about 70%.

When the organic solvents extracting pravastatin and analogues thereof from the concentrated filtrate are ethyl acetate, n-propyl acetate and n-butyl acetate, the purity ofpravastatin sodium (%) is as follows. From the result shown below, it is clear that highly pure pravastatin is obtained by extraction with n-propyl acetate or n-butyl acetate rather than extraction with ethyl acetate.

**Table 1**

| | Example (1a) | Example 2 | Comparative Example 1 |
|---|---|---|---|
| Extraction solvent | n-butyl acetate | n-propyl acetate | ethyl acetate |
| Purity of pravastatin (%) | >90 | >85 | about 70 |

## Claims

1. A composition comprising pravastatin sodium which is industrially produced and contains a compound of formula (I) the quantity of which is 0.1% or less by weight of the quantity of pravastatin sodium.

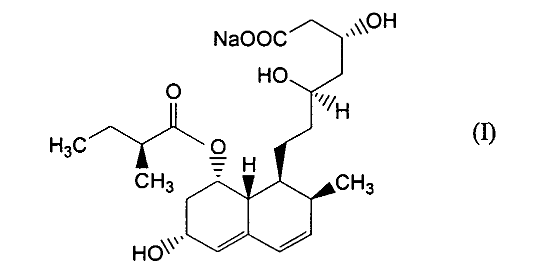

2. A composition comprising pravastatin sodium according to claim 1, which is industrially produced and contains 99.5% or more of pravastatin sodium.
